# FASCICULE DE BREVET EUROPEEN

(11) **EP 1 265 585 B1**
(45) Date de publication et mention de la délivrance du brevet: **14.06.2006**
(21) Numéro de dépôt: 01917191.7
(22) Date de dépôt: 20.03.2001
(51) Int. Cl.: A61K 8/06, A61K 8/89

(54) **EMULSION COMPRENANT UN SYSTEME HUILE SILICONE / CO-SOLVANT CONCENTRE EN MATIERE(S) ACTIVE(S) COSMETIQUE(S) LIPOSOLUBLE(S) ET FORMULATIONS COSMETIQUES CORRESPONDANTES**
EMULSION ENTHALTEND SILICONÖL- UND CO-LÖSUNGSMITTELSYSTEM ENHALTEND FETTLÖSLICHE KOSMETISCHE WIRKSTOFFE UND DARAUS HERGESTELLETEN KOSMETISCHEN ZUBEREITUNGEN
EMULSION COMPRISING A SILICONE OIL/COSOLVENT SYSTEM CONCENTRATED IN ACTIVE LIPOSOLUBLE COSMETIC SUBSTANCE(S) AND CORRESPONDING COSMETIC FORMULATIONS

(30) Priorité: 22.03.2000 FR 0003677
(43) Date de publication de la demande: 18.12.2002
(73) Titulaire: RHODIA CHIMIE, 92512 Boulogne Billancourt Cedex (FR)
(72) Inventeur: MERCIER, Jean-Michel, F-94320 Thiais (FR); RICCA, Jean-Marc, Singapour 248648 (SI); MARTIN, Nadia, F-69006 Lyon (FR)
(74) Mandataire: Bernasconi, Jean Raymond
(86) Numéro de dépôt international: PCT/FR2001/000831
(87) Numéro de publication internationale: WO 2001/070177

(56) Documents cités:
- EP-A- 0 152 953
- EP-A- 0 437 216
- EP-A- 0 576 748
- US-A- 5 068 099
- US-A- 5 298 236

## Description

La présente invention a pour objet une émulsion comprenant un concentré de matières actives dans un dérivé de silicone et ses utilisations dans des formulations cosmétiques.

Au sens de l'invention, on entend par formulation cosmétique, tous les produits ou préparations cosmétiques du type de ceux ou celles décrit(e)s dans l'annexe I ("Illustrative list by category of cosmetic products") de la directive européenne n° 76/768/CEE du 25 juillet 1976, dite Directive Cosmétique.

De manière générale, les compositions cosmétiques sont formulées sous la forme d'un grand nombre de type de produits destinés à être appliqués soit sur le cheveu, comme les mousses, les gels (coiffant notamment), les conditionneurs, les formulations pour le coiffage ou pour faciliter le peignage et/ou le démêlage des cheveux, les formules de rinçage, soit sur la peau comme les lotions pour les mains et le corps, les produits régulant l'hydratation de la peau, les laits de toilette, les compositions démaquillantes, les produits dépilatoires, les crèmes ou lotions de protection contre le soleil et les rayonnements ultraviolets, les crèmes de soin, les préparations anti-acnée, les analgésiques locaux, les formulations maquillage de type mascaras, fonds de teint, vernis à ongles les produits destinés à être appliqués sur les lèvres ou autres muqueuses, les sticks, les compositions solides de type savon de toilette ainsi que d'autres formulations du même type.

Les compositions cosmétiques ou dermatologiques se présentent, pour la plupart, sous une forme liquide épaissie de type gel ou crème. Ce type de présentation, approprié pour le consommateur, correspond le plus souvent à une préoccupation pratique pour le formulateur : faciliter la prise du produit hors de son conditionnement sans perte significative, limiter la diffusion du produit à la zone locale de traitement, pouvoir répartir le produit de façon régulière sur la zone locale de traitement et pouvoir l'utiliser dans des quantités suffisantes pour obtenir l'effet cosmétique ou dermatologique recherché.

Cet objectif est primordial pour les formulations telles que celles des produits pour le soin, l'hygiène ou le maquillage qui doivent bien se répartir de façon homogène sur la surface locale à traiter ainsi que les compositions capillaires qui doivent s'étaler et se répartir de façon régulière le long des fibres kératiniques.

Les dérivés de silicone constituent tout particulièrement des véhicules de choix pour transporter et/ou délivrer à travers la peau et/ou les fibres kératiniques un certain nombre de produits actifs cosmétiques ou pharmaceutiques. Leur perméabilité aux transferts gazeux, à la vapeur d'eau en particulier, leur caractère « amphipatique induit » avec une localisation en surface, combinés à leur innocuité expliquent en particulier leur succès dans le domaine cosmétique.

Toutefois, l'encapsulation des actifs cosmétiques au sein de ce type de formulations peut dans certains cas soulever des difficultés. Les actifs cosmétiques peuvent, d'une part, être difficiles à incorporer dans la formulation, pour des raisons notamment d'insolubilité, et, d'autre part, y être instables et/ou incompatibles avec d'autres ingrédients. Cette instabilité se traduit généralement par des phénomènes de démixtion, d'émulsion ou de cristallisation au niveau de la formulation cosmétique.

La solution généralement retenue pour prévenir la manifestation de ces phénomènes secondaires indésirables, consiste à limiter la quantité en actifs dans lesdites formulations.

EP-A-0576748 décrit une émulsion comprenant une phase aqueuse et une phase huileuse pouvant comprendre une silicone. Un solvant tel que l'éthanol peut être ajouté à l'émulsion après préparation.

La demande EP-A-0152953 décrit un composé de soin personnel comprenant une matière active liposoluble. Il s'agit d'une émulsion eau dans silicone.

La demande EP-A-0467216 décrit une formulation comprenant un solvant, une silicone et des esters de sorbitol.

La présente invention a précisément pour objet de proposer une émulsion huile dans eau comprenant à titre de phase huileuse un système silicone / co-solvant permettant avantageusement l'encapsulation jusqu'à 100% en poids en matières actives organiques liposolubles par rapport au poids en silicone. Cette émulsion est particulièrement intéressant pour préparer des formulations cosmétiques à teneur élevée en matières actives.

Avantageusement, les émulsions et/ou formulations cosmétiques correspondantes présentent une stabilité satisfaisante et ne sont pas sujettes aux phénomènes de démixtion et/ou cristallisation évoqués ci-dessus.

Un premier aspect de l'invention concerne donc une émulsion huile dans eau comprenant à titre de phase huileuse un système silicone / co-solvant / matière(s) active(s), caractérisé en ce qu'il comprend sous une forme solubilisée au moins 25% en poids par rapport au poids en silicone d'au moins une matière active liposoluble et naturellement non soluble dans ledit silicone.

De manière inattendue, les inventeurs ont mis en évidence qu'il est possible, grâce à l'utilisation d'un co-solvant adéquat, d'incorporer, de manière efficace et stable, des quantités importantes en matières actives cosmétiques dans un dérivé de silicone et via ce système dans des émulsions et/ou formulations cosmétiques. Ces systèmes peuvent ainsi avantageusement contenir jusqu'à 100 % en poids en matières actives.

Les silicones pouvant être utilisées dans le cadre de la présente invention sont de préférence des huiles ou gommes de silicone et plus particulièrement des huiles non volatiles.

Si l'on se réfère à l'espace tridimensionnel de solubilité, dans lequel prennent place les solvants et toutes les molécules organiques, est défini par CM Hansen dans "The three dimensionnal solubility parameters" J. Paint Technol. 39, 105 (1967), il s'avère particulièrement avantageux selon l'invention d'utiliser les silicones présentant un δ_{D} inférieur à environ 15, δ_{P} inférieur à environ 1 et δ_{H} inférieur à environ 5, c'est-à-dire non propices naturellement à la dissolution de matières actives notamment très polaires; δ_{D}, δ_{P} et δ_{H} représentent les paramètres de solubilité partiels reliés respectivement aux forces de dispersion de London, de polarité de Keesom et de liaison hydrogène, sachant que ces paramètres partiels sont les composants du paramètre de solubilité global δ, dit de Hildebrand, relié à la cohésion volumique de la molécule.

En outre, les silicones conformes à l'invention présentent de préférence une viscosité supérieure à 300 mPa.s de préférence supérieure à 10.000 mPa.s et de manière plus préférentielle à 100 000 mPa.s.

Les huiles silicones dites de forte viscosité présentent une viscosité supérieure à 1000 mPa.s, par exemple supérieure à 3000 mPa.s, de préférence supérieure à 5000 mPa.s ou jusqu'à 500 000 mPa.s. Les silicones de faible viscosité sont définis comme présentant une viscosité inférieure à 1000 mPa.s, et de préférence comprise entre 1 et 1000 mPa.s, par exemple entre 20 et 1000 mPa.s.

Dans le cadre de l'invention et à défaut de précisions contraires, les valeurs des viscosités sont les valeurs des viscosités dynamiques mesurées à 25°C à l'aide d'un viscosimètre BROOKFIELD selon les indications de la norme AFNOR NFT 76102.

Plus particulièrement, les silicones mis en oeuvre selon l'invention peuvent être constitués en tout ou partie de motifs de formule :

R'₃₋ₐRₐSiO_{1/2} (motif M) et/ou R₂SiO (motif D)

formules où :
- a est un entier de 0 à 3
- les radicaux R sont identiques ou différents et représentent :
   - un groupe hydrocarboné aliphatique saturé ou insaturé contenant de 1 à 10 atomes de carbone ;
   - un groupe hydrocarboné aromatique contenant de 6 à 13 atomes de carbone ;
   - un groupe organique polaire lié au silicium par une liaison Si-C ou Si-O-C ;
   - un atome d'hydrogène :
      - les radicaux R' sont identiques ou différents et représentent
   - un groupe OH
   - un groupe alcoxy ou alcényloxy contenant de 1 à 10 atomes de carbone ;
   - un groupe aryloxy contenant de 6 à 13 atomes de carbone ;
   - un groupe acyloxy contenant de 2 à 13 atomes de carbone ;
   - un groupe cétiminoxy contenant de 3 à 8 atomes de carbone ;
   - un groupe amino- ou amido-fonctionnel contenant de 1 à 6 atomes de carbone, lié au silicium par une liaison Si-N ;

De préférence au moins 80% des radicaux R desdites huiles représentent un groupe méthyle.

Ces silicones peuvent éventuellement comprendre de préférence 5 % des motifs de formules T ou Q :

RSiO_{3/2} (motif T) et/ou SiO₂ (motif Q)

formule dans laquelle R a la définition donnée ci-dessus.

A titre d'exemples de radicaux hydrocarbonés aliphatiques ou aromatiques R, on peut citer les groupes :
- alkyle, de préférence alkyle en C₁-C₁₀ éventuellement halogéné, tels que méthyle, éthyle, octyle, trifluoropropyle ;
- alcoxyalkylène, de préférence en C₂-C₁₀, mieux encore en C₂-C₆, tels que -CH₂-CH₂-O-CH₃;
- alcényles, de préférence alcényle en C₂-C₁₀, tels que vinyle, allyle, hexényle, décényle, décadiényle ;
- alcényloxyalkylène tels que -(CH₂)₃-O-CH₂-CH=CH₂, ou alcényloxyalcoxyalkyle tels que -(CH₂)₃-OCH₂-CH₂-O-CH=CH₂ dans lesquels les parties alkyle sont de préférence en C₁-C₁₀ et les parties alcényles sont de préférence en C₂-C₁₀;
- aryles, de préférence en C₆-C₁₃, tels que phényle.

A titre d'exemples de groupes organiques polaires R, on peut citer les groupes :
- hydroxyfonctionnels tels que des groupes alkyle substitués par un ou plusieurs groupes hydroxy ou di(hydroxyalkyl)amino et éventuellement interrompu par un ou plusieurs groupes bivalents hydroxyalkylamino. Par alkyle on entend une chaîne hydrocarbonée de préférence en C₁-C₁₀, mieux encore en C₁-C₆; des exemples de ces groupes sont -(CH₂)₃-OH -(CH₂)₄N(CH₂CH₂OH)₂ ; -(CH₂)₃-N(CH₂CH₂OH)-CH₂-CH₂₋N(CH₂CH₂OH)₂ :
- aminofonctionnels tels que alkyle substitué par un ou plusieurs groupes amino ou aminoalkylamino où alkyle est tel que défini ci-dessus ; des exemples en sont -(CH₂)₃-NH₂ ; (CH₂)₃-NH-(CH₂)₂NH₂ ;
- amidofonctionnels tels que alkyle substitué par un ou plusieurs groupes acylamino et éventuellement interrompu par un ou plusieurs groupes bivalents alkyl-CO-N< où alkyle est tel que défini ci-dessus et acyle représente alkylcarbonyle ; un exemple est le groupe -(CH₂)₃-N(COCH₃)-(CH₂)₂NH(COCH₃) ;
- carboxyfonctionnels tels que carboxyalkyle éventuellement interrompu par un ou plusieurs atomes d'oxygène ou de soufre où alkyle est tel que défini ci-dessus ; un exemple est le groupe -CH₂-CH₂-S-CH₂-COOH.

A titre d'exemples de radicaux R', on peut citer les groupes :
- alcoxy, de préférence en C₁-C₁₀. mieux encore en C₁-C₆, tels que méthoxy, éthoxy, octyloxy ;
- alcényloxy de préférence en C₂-C₁₀, mieux encore en C₂-C₆ ;
- aryloxy de préférence en C₆-C₁₃, tels que phényloxy ;
- acyloxy où acyle est de préférence (C₁-C₁₂)alkylcarbonyle tels que acétoxy ;
- cétiminoxy contenant de 3 à 8 atomes de carbone tels que ON=C(CH₃)C₂H₅ ;
- aminofonctionnels tels que alkyle ou aryle substitué par amino, alkyle étant de préférence en C₁-C₆ et aryle désignant un groupe aromatique cyclique hydrocarboné de préférence en C₆-C₁₃ tel que phényle ; des exemples en sont éthylamino, phénylamino ;
- amidofonctionnels tels que alkylcarbonylamino où alkyle est de préférence en C₁-C₆ des exemples en sont méthylacétamido.

A titre d'exemples concrets de "motifs D" on peut citer : (CH₃)₂SiO; CH₃(CH=CH₂)SiO ; CH₃(C₆H₅)SiO ; (C₆H₅)₂SiO ; CH₃HSiO; CH₃(CH₂-CH₂₋CH₂OH)SiO.

A titre d'exemples concrets de "motifs M", on peut citer : (CH₃)₃SiO_{1/2} : (CH₃)₂(OH)SiO_{1/2} ; (CH₃)₂(CH=CH₂)SiO_{1/2} ; (CH₃)₂HSiO_{1/2} : (OCH₃)₃SiO_{1/2} : [O-C(CH₃)=CH₂]₃SiO_{1/2} : [ON=C(CH₃)]₃SiO_{1/2}; (NH-CH₃)₃SiO_{1/2}; (NH-CO-CH₃)₃SiO_{1/2}.

A titre d'exemples concrets de "motifs T", on peut citer : CH₃SiO_{3/2}; (CH=CH₂)SiO_{3/2} ; HSiO_{3/2}.

Lorsque les silicones contiennent des radicaux R réactifs et/ou polaires (tels que H, OH, vinyle, allyle, héxényle, aminoalkyles ....), ces derniers ne représentent généralement pas plus de 5% du poids et de préférence pas plus de 1 % du poids de silicone.

Les huiles et gommes polydiméthylsiloxanes et α,ω-bis(hydroxy)polydiméthylsiloxanes ainsi que les gommes polydiméthylsiloxanes, polyphénylméthylsiloxane et α,ω-bis(hydroxy)polydiméthylsiloxanes sont des produits du commerce bien connus.

On préfère plus particulièrement les huiles et gommes α,ω-bis(triméthyl)polydiméthylsiloxanes ; les huiles et les gommes α,ω-bis(hydroxy)polydiméthylsiloxanes.

Des exemples préférés d'huiles polydiméthylsiloxane sont celles dont la chaîne de polydiméthylsiloxane est bloquée à ses deux extrémités par un groupe (CH₃)₃SiO_{1/2} ou (CH₃)₂(OH)SiO_{1/2} de viscosité dynamique de 20 mPa.s, de 350 mPa.s, de 750 mPa.s, de 80000 mPa.s ou de 500000 mPa.s.

A titre représentatif des huiles silicones convenant tout particulièrement à la présente invention, on peut notamment citer les silicones de type polydiméthylsiloxane (diméthicone) et diphényldiméthicone.

Outre le silicone tel que défini ci-dessus, on peut envisager d'incorporer une ou plusieurs huiles complémentaires, de préférence choisies parmi :
- les huiles minérales telles que l'huile de paraffine et de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de Purcellin, les isoparaffines,
- les huiles fluorées et perfluorées.

Ces huiles complémentaires sont mises en oeuvre en quantité telle qu'elles n'ont pas d'incidence en terme de polarité comparativement au silicone retenu.

Conformément à la présente invention, le silicone est formulé avec un co-solvant.

Le co-solvant est choisi de manière à permettre la solubilisation de la matière active dans le silicone et plus précisément de manière à suppléer à l'incompatibilité naturelle manifestée en termes de solubilité entre le silicone et la matière active.

A ces fins, le co-solvant est généralement choisi de manière à présenter dans l'espace de solubilité de Hansen les paramètres suivants :
δ_{D} d'interactions London variant de 11 à 17 (J/cm³)^{1/2},
δ_{P} d'interactions Keesom supérieur à 2,5 (J/cm³)^{1/2}, et
δ_{H} de liaison hydrogène allant de 0 à 23 (J/cm³)^{1/2}.

Selon une variante préférée de l'invention, ce co-solvant est un ester aliphatique d'alkyle en C₁₀ à C₂₀ et de préférence un néopentanoate d'alkyle en C₅ à C₁₂ et de préférence d'isodécyle.

Est notamment préféré selon l'invention un système comprenant du néopentanoate d'isodécyle et un polydiméthylsiloxane de viscosité égale à 500 000 mPa.s.

A titre représentatif de système conforme à la présente invention, on peut plus particulièrement citer celui associant à un polydiméthylsiloxane de type diméthicone ou diphényldiméthicone, le néopentanoate d'isodécyle. Les deux solvants sont de préférence utilisés dans un rapport pondéral de néopentanoate d'isodécyle pour un polyméthylsiloxane variant de 3 :2 à 3 :1.

Les matières actives liposolubles peuvent être solides ou liquides. Le choix est bien entendu effectué en fonction de la formulation cosmétique correspondante et de son site d'application à savoir cutané, capillaire ou buccodentaire.

Lorsque les formulations cosmétiques correspondantes sont destinées à un usage buccodentaire, le matériau actif peut être choisi dans le groupe consistant en les composés anti-caries, les composés anti-microbiens, les composés anti-plaques, les arômes et leurs mélanges.

Les arômes peuvent être notamment des huiles essentielles, telles huile de menthe, huile de citron, huile d'anis par exemple. Les agents anti-microbiens peuvent être choisis parmi le thymol, le menthol, le triclosan, le 4-hexylrésorcinol, le phénol, l'eucalyptol, l'acide benzoïque, le peroxyde benzoïque, le parabène de butyle, et leurs mélanges.

Lorsque le site d'application de la formulation cosmétique est la peau, les cheveux ou les ongles, les matières actives appropriées peuvent être des composés anti-vieillissement, des vitamines, des parfums, des agents anti-microbiens, des bactéricides, des agents absorbeurs d'UV, des agents anti-acné, des agents anti-cellulite, ainsi que des mélanges de ceux-ci.

A titre représentatif des agents anti-vieillissement, on peut notamment citer les rétinoïdes, les acides α- et β- hydroxy, leurs sels et leurs esters, les vitamines liposolubles, le palmitate d'ascorbyle, les céramides, les pseudo-céramides, les phospholipides, les acides gras, les alcools gras, le cholestérol, les stérols et leurs mélanges. Comme acides gras et alcools préférés, on peut plus particulièrement citer ceux possédant des chaînes alkyles, linéaires ou ramifiées contenant de 12 à 20 atomes de carbone. Il peut notamment s'agir d'acide linoléique.

Comme vitamines peuvent être formulées la vitamine A et ses dérivés, la vitamine B2, l'acide pantothénique, la vitamine D et la vitamine E.

A titre représentatif des matériaux absorbant UV, on peut plus particulièrement citer les dérivés aminobenzoate de type PABA et PARA, les salicylates, les cinnamates, les anthranilates, les dibenzoylméthanes, les dérivés du camphre et leurs mélanges.

Comme agents anti-cellulite, conviennent notamment l'isobutylméthylxanthine et la théophyline.

Enfin, à titre représentatif des agents anti-acné, on peut notamment citer le résorcinol, l'acétate de résorcinol, le peroxyde benzoyle, l'acide salicylique, l'acide azélaique, les acides dicarboxyliques à longue chaîne et de nombreux composés naturels.

Cette matière active peut être bien entendu également des extraits marins, des extraits végétaux, des parfums, des agents colorants ou des particules minérales à l'état nanoparticulaire comme l'oxyde de zinc, le dioxyde de titane ou les oxydes de cérium.

Il peut également s'agir de mélanges des matières actives identifiées ci-dessus.

La ou les matière(s) active(s) retenue(s) sont solubilisées dans le silicone ou de préférence dans le co-solvant. Le second réactif, à savoir le co-solvant ou le dérivé silicone est ensuite ajouté et l'ensemble homogénéisé sous agitation. En fait, le choix du milieu de solubilisation est généralement effectué en fonction de la concentration en matière active recherchée.

Le mélange est généralement réalisé à température ambiante. Toutefois, il peut, le cas échéant, être réalisé à une température plus élevée pour faciliter la solubilisation dans la mesure où cette température ne risque pas d'affecter les propriétés de la matière active.

La présente invention vise dans un second aspect l'utilisation d'une émulsion de type huile dans eau et comprenant à titre de phase huileuse, un système huile de silicone / co-solvant tel que défini précédemment pour la préparation d'une émulsion et/ou une formulation de préférence cosmétique concentrée en matière(s) active(s) liposoluble(s) et de préférence comprenant au moins 25% en poids et avantageusement jusqu'à 100% en poids en matières actives par rapport au poids en silicone.

La préparation de ce type d'émulsion est connue de l'homme du métier. Plusieurs méthodes existent et sont décrites notamment dans les références suivantes : Understanding Emulsions (Randy Schueller and Perry Romanowski Cosmetic & Toiletries Vol 113 Sep 98), Formulating Cosmetic Emulsions (Dr Gillian M. Eccleston, Cosmetic & Toiletries Vol 112 Dec 97) et Becher's Encyclopedia of Emulsion Technology (P. Becher Ed M. Dekker 1983 et 1985).

L'émulsion revendiquée est stabilisée à l'aide d'un ou plusieurs tensioactifs.

La nature du tensioactif utilisable pour l'émulsification n'est pas critique dans la mesure où il est compatible avec un usage cosmétique.

Ainsi, on peut utiliser des tensioactifs non ioniques, cationiques, anioniques ou même zwitterioniques.

Des exemples de tensioactifs anioniques sont :
- les alkylesters sulfonates de formule R-CH(SO₃M)-COOR', où R représente un radical alkyle en C₈-C₂₀, de préférence en C₁₀-C₁₆, R' un radical alkyle en C₁-C₆, de préférence en C₁-C₃ et M un cation alcalin (sodium, potassium, lithium), ammonium substitué ou non substitué (méthyl-, diméthyl-, triméthyl-, tétraméthylammonium, diméthylpipéridinium ...) ou dérivé d'une alcanolamine (monoéthanolamine, diéthanolamine, triéthanolamine ...). On peut citer tout particulièrement les méthyl ester sulfonates dont le radical R est en C₁₄-C₁₆;
- les alkylsulfates de formule ROSO₃M, où R représente un radical alkyle ou hydroxyalkyle en C₁₀-C₂₄, de préférence en C₁₂-C₂₀ et tout particulièrement en C₁₂-C₁₈, M représentant un atome d'hydrogène ou un cation de même définition que ci-dessus, ainsi que leurs dérivés éthoxylénés (OE) et/ou propoxylénés (OP), présentant en moyenne de 0,5 à 6 motifs, de préférence de 0,5 à 3 motifs OE et/ou OP ;
- les alkylamides sulfates de formule R₁CONHR'₁OSO₃M où R₁ représente un radical alkyle en C₂-C₂₂, de préférence en C₆-C₂₀, R'₁ un radical alkyle en C₂-C₃, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;
- les sels d'acides gras saturés ou insaturés en C₈-C₂₄, de préférence en C₁₄-C₂₀, les alkylbenzènesulfonates en C₉-C₂₀, les alkylsulfonates primaires ou secondaires en C₈-C₂₂, les alkylglycérol sulfonates, les acides polycarboxyliques sulfonés décrits dans GB-A-1 082 179, les sulfonates de paraffine, les N-acyl N-alkyltaurates, les alkylphosphates, les alkyliséthionates, les alkylsuccinamates et alkylsulfosuccinates, les monoesters ou diesters de sulfosuccinates, les N-acyl sarcosinates, les sulfates d'alkylglycosides, les polyéthoxycarboxylates, le cation étant un métal alcalin (sodium, potassium, lithium), un reste ammonium substitué ou non substitué (méthyl-, diméthyl-, triméthyl-, tétraméthylammonium, diméthylpipéridinium ...) ou dérivé d'une alcanolamine (monoéthanolamine, diéthanolamine, triéthanolamine ...) ; et
- les phosphates ou alkylphosphates esters.

Des exemples de tensioactifs non ioniques sont :
- les alkylphénols polyoxyalkylénés (polyéthoxyéthylénés, polyoxypropylénés, polyoxybutylénés) dont le substituant alkyle est en C₆₋C₁₂ et contenant de 5 à 25 motifs oxyalkylènes ; à titre d'exemple, on peut citer les TRITON X-45, X-114, X-100 ou X-102 commercialisés par Rohm & Haas Cy. ;
- les glucosamines, glucamides ;
- les glycérolamides dérivés de N-alkylamines (US-A-5,223,179 et FR-A-1,585,966) ;
- les alcools aliphatiques en C₈-C₂₂ polyoxyalkylénés contenant de 1 à 25 motifs oxyalkylènes (oxyéthylène, oxypropylène) ; à titre d'exemple, on peut citer les TERGITOL 15-S-9, TERGITOL 24-L-6 NMW commercialisés par Union Carbide Corp., NEODOL 45-9, NEODOL 23-65, NEODOL 45-7, NEODOL 45-4 commercialisés par Shell Chemical Cy., KYRO EOB commercialisé par The Procter & Gamble Cy.
- les produits résultant de la condensation de l'oxyde d'éthylène avec un composé hydrophobe résultant de la condensation de l'oxyde de propylène avec le propylène glycol, tels les PLURONIC® commercialisés par BASF ;
- les oxydes d'amines tels que les oxydes d'alkyl C₁₀-C₁₈ diméthylamines, les oxydes d'alkoxy C₈-C₂₂ éthyldihydroxyéthylamines ;
- les alkylpolyglycosides décrits dans US-A-4 565 647 et leurs dérivés polyoxyalkylénés ;
- les amides d'acides gras en C₈-C₂₀;
- les acides gras éthoxylés ; et
- les amides, amines, amidoamines éthoxylées ;
- les esters de sorbitane polyoxyalkyléné ou non avec des acides gras en C₈-C₂₀ tels que les oléates de sorbitane polyoxyéthyléné (par exemple SPAN® et TWEEN®) ;
- les esters du saccharose avec des acides gras en C₈-C₂₀;
- les mélanges d'esters du saccharose avec des acides gras en C₈₋C₂₀ avec des mono-, di- et/ou tri-glycérides d'acide gras en C₈-C₂₀.

Des exemples de tensioactifs amphotères et zwitterioniques sont :
- ceux de type bétaïne comme les bétaïnes, les sulfo-bétaïnes, les amidoalkylbétaïnes, les sulfo-bétaïnes, les alkylsultaines, les alkyltriméthylsulfobétaïnes,
- les produits de condensation d'acides gras et d'hydrolysats de protéines,
- les alkylampho-propionates ou -dipropionates,
- les dérivés amphotères des alkylpolyamines comme l'AMPHIONIC XL® commercialisé par RHONE-POULENC, AMPHOLAC 7T/X® et AMPHOLAC 7C/X® commercialisés par BEROL NOBEL, et
- les cocoamphoacétates et cocoamphodiacétates.

Les tensioactifs non ioniques sont néanmoins préférés et plus particulièrement les tensioactifs non ioniques du type alcool gras à chaîne linéaire ou ramifiée polyoxyalkylénés résultant de la condensation d'un alcool gras avec un oxyde d'alkylène en C₂-C₄ tel que oxyde d'éthylène, oxyde de propylène ou oxyde de butylène. A titre d'exemple, on peut citer l'alcool isotridécylique éthoxylé.

Plus généralement, les tensioactifs non ioniques préférés sont ceux dont le HLB est compris entre 8 et 16, préférablement entre 10 et 15, mieux encore entre 12,5 et 15.

Le terme HLB (Hydrophilic Lipophilic Balance) désigne le rapport de l'hydrophilie des groupements polaires des molécules de tensioactifs à l'hydrophobie de leur partie lipophile. Des valeurs de HLB sont notamment rapportées dans différents manuels de base tels que le "Handbook des excipients pharmaceutiques, The Pharmaceutical Press, London, 1994".

Le tensioactif nécessaire à la préparation de l'émulsion est soit présent en totalité dans la phase aqueuse, soit présent en totalité dans la phase huileuse, soit encore réparti, dans des proportions variables, entre ces deux phases.

Généralement, l'émulsion revendiquée est constituée au moins de 50% en poids et de préférence d'au moins 70% en poids en un système selon l'invention.

L'émulsion revendiquée présente de préférence une taille de gouttelettes comprise entre 25 nm et 350 µm, avantageusement inférieure à 3 µm et plus préférentiellement inférieure à 0,3 µm,

Un quatrième aspect de la présente invention concerne une formulation cosmétique comprenant au moins une émulsion conforme à la présente invention.

De préférence, les formulations cosmétiques conformes à l'invention, font appel à un véhicule, généralement de l'eau, ou à un mélange de plusieurs véhicules, présent dans lesdites formulations à des concentrations comprises entre 0,5 et 99,5 % environ et plus préférentiellement entre 5 et 90 % environ.

Les véhicules compatibles avec les formulations selon l'invention comprennent par exemple ceux utilisés dans les spray, les mousses, les toniques, les gels, les shampooings, ou encore les lotions de rinçage.

Bien entendu le choix du véhicule approprié dépend de l'application spécifique visée par la formulation. Un véhicule convenant pour une formulation destinée à demeurer sur la surface de laquelle elle a été appliquée (par exemple spray, mousse, lotion, tonique ou gel), ne sera pas le véhicule approprié pour une formulation devant être rincée après utilisation (par exemple shampooing conditionneur, lotion de rinçage).

Les véhicules susceptibles d'être utilisés peuvent donc être simples ou complexes et inclure un grand nombre de produits habituellement utilisés dans les formulations cosmétiques destinées à un usage capillaire, cutané ou solaire.

Il peut ainsi s'agir d'eau complémentée éventuellement en un solubilisant pour dissoudre ou disperser les principes actifs utilisés, tels que les alcools en C₁-C₆, et leurs mélanges, en particulier éthanol, isopropanol ou propylèneglycol, et leurs mélanges.

Lorsque les formulations cosmétiques se présentent sous la forme de sprays, lotions, toniques, gels, ou mousses, les solvants préférentiels comprennent l'eau, l'éthanol, les dérivés volatils de silicone, et leurs mélanges. Les solvants utilisés dans ces mélanges peuvent être miscibles ou non miscibles les uns avec les autres. Les mousses et les sprays aérosol peuvent aussi utiliser n'importe quel propulseur capable de générer les produits sous forme de mousse ou de sprays fins, uniformes. A titre d'exemples, on peut citer le diméthyléther, le propane, le n-butane, ou l'isobutane.

Parallèlement aux véhicules identifiés précédemment, les formulations cosmétiques selon l'invention peuvent contenir des agents tensioactifs, mis en oeuvre pour disperser, solubiliser, stabiliser divers composés annexes. Ces agents tensioactifs peuvent être de type anionique, non-ionique, cationique, zwitterionique ou amphotère et sont de préférence choisis parmi les tensioactifs identifiés précédemment.

Comme il ressort de ce qui précède, les formulations cosmétiques conformes à la présente invention ont pour vocation principale de délivrer et de déposer sur des surfaces cibles, cutanées ou capillaires, par exemple la matière active qui est formulée. Cette formulation doit donc permettre le dépôt au niveau de la surface traitée de la matière active qui doit y demeurer après que la formulation en soit éliminée par lavage et son rinçage. Dans la mesure où ces surfaces de dépôts sont chargées négativement, l'approche habituelle pour procéder à ce dépôt consiste à associer à la matière active des composés cationiques et plus particulièrement des polymères cationiques.

Les polymères cationiques plus particulièrement utiles pour assurer ce rôle de conditionneur sont notamment des polymères de type polyquaternium, comme par exemple les polyquaternium-1, polyquatemium-2, polyquatemium-4, polyquatemium-5, polyquatemium-6 (également connu comme Merquat 100® disponible auprès de Calgon), polyquatemium-7 (également connu comme Merquat 550® disponible auprès de Calgon), polyquaternium-8, polyquatemium-9, polyquaternium-10 (également connu comme Polymer JR 400®), polyquaternium-11, polyquaternium-12, polyquaternium-13, polyquaternium-14, polyquatemium-15, polyquatemium-16, polyquatemium-17, polyquatemium-18, polyquaternium-19, polyquatemium-20, polyquaternium-24, polyquatemium-27, polyquatemium-28, polyquatemium-29 (également connu comme Kytamer KC® disponible auprès de Amerchol), polyquaternium-30, polyquaternium-31, polyquaternium-32, polyquaternium-33, polyquatemium-34, polyquaternium-35, polyquatemium-36, polyquatemium-37, polyquatemium-37.

Conviennent également les dérivés cationiques de polysaccharides, comme la cellulose cocodimonium hydroxyéthyl, le guar hydroxypropyl trimonium chlorure, l'hydroxypropyl guar hydroxypropyl trimonium chlorure (JAGUAR C 13S®, JAGUAR C162® commercialisés par RHODIA), l'éther de poly(oxyéthanediyl-1,2)hydroxy-2 chlorure de triméthylammonium-3 propyl cellulose ou polyquaternium-10.

Les formulations cosmétiques peuvent également contenir des polymères présentant des propriétés filmogènes pouvant être utilisés pour apporter une fonction fixante. Ces polymères sont généralement présents à des concentrations comprises entre 0,01 et 10 %, préférentiellement entre 0,5 et 5 %. Ils sont préférentiellement du type polyvinylpyrrolidone, copolymères de polyvinylpyrrolidone et de méthyl méthacrylate, copolymère de polyvinylpyrrolidone et d'acétate de vinyle, copolymères polytéréphtalate d'éthylène glycol/polyéthylène glycol, polymères copolyesters téréphtaliques sulfonés.

Les formulations cosmétiques peuvent également contenir des dérivés polymériques exerçant une fonction protectrice, en quantités de l'ordre de 0,01-10 %, de préférence environ 0,1-5 % en poids, dérivés tels que les dérivés cellulosiques, les polyvinylesters greffés sur des squelettes polyalkylènes, les alcools polyvinyliques, les polymères copolyesters téréphtaliques sulfonés, les monoamines ou polyamines éthoxylées et les polymères d'amines éthoxylées.

On peut également incorporer aux formulations cosmétiques des agents hydratants. A titre illustratif de ces derniers, on peut notamment citer le glycérol, le propylène glycol, l'urée, le collagène, la gélatine, et des émollients qui sont généralement choisis parmi les alkylmonoglycérides, les alkyldiglycérides, les triglycérides comme les huiles extraites des plantes et des végétaux ou leurs dérivés hydrogénés, les huiles minérales ou les huiles paraffiniques, les diols, les esters gras, les silicones.

Des agents conservateurs comme les esters de l'acide p-hydroxybenzoïque, le benzoate de sodium, ou tout agent chimique évitant la prolifération bactérienne ou des moisissures et utilisé traditionnellement dans les compositions cosmétiques sont généralement introduits dans ces compositions à hauteur de 0,01 à 3 % en poids conformément à l'annexe VII de la réglementation cosmétique.

Finalement, les formulations cosmétiques peuvent aussi contenir des polymères viscosants ou gélifiants comme les polyacrylates réticulés de type CARBOPOL® commercialisés par BF GOODRICH, les copolymères acryliques anioniques de type ACULYN® commercialisés par ISP (International Specialty Products), les dérivés de la cellulose comme l'hydroxypropylcellulose, la carboxyméthylcellulose, les guars et leurs dérivés comme l'hydroxypropyl guar tel que Jaguar HP®, la caroube, la gomme de tara ou de cassia, la gomme xanthane tel que le Rhodicare®, les succinoglycanes, les alginates, les carraghénannes, les dérivés de la chitine ou tout autre polysaccharide à fonction texturante.

Les formulations cosmétiques selon l'invention peuvent être avantageusement mises en oeuvre dans les domaines bucco-dentaire, capillaire, solaire, soin corporel et du maquillage.

Les exemples soumis ci-après, sont présentés simplement à titre illustratif et non limitatif de la présente invention.

### EXEMPLES 1 A 5

### Préparation de concentré en matières actives cosmétiques dans une huile de silicone.

On utilise à titre de co-solvant le néopentanoate d'isodécyle, et d'huile de silicone le polydiméthylsiloxane 5 000 000 commercialisé par RHODIA.

Le ou les actif(s) est/sont incorporé(s) au préalable dans le co-solvant puis la silicone est homogénéisée avec le mélange résultant.

Les quantités en réactifs et la nature des actifs incorporés sont présentées dans le tableau 1 ci-après.

**TABLEAU 1.**

| | Mélange Actif | Exemple 1 | Exemple 2 | Exemple 3 | Exemple 4 | Exemple 5 |
|---|---|---|---|---|---|---|
| Co-solvant | Néopentanoate d'iso-décyle | 55 | 70.5 | 64 | 50 | 58.8 |
| | Octyl-methoxy cinnamate | 17.3 | - | 9.3 | - | - |
| Matières actives | Butyl methoxydibenzoyl Methane | - | 6 | 5.5 | - | - |
| | Drometrizole trisiloxane | - | - | - | 25 | - |
| | Triclosan | - | - | - | - | 11.8 |
| Huile de silicone | PDMS 500000 | 27.7 | 23.5 | 21.2 | 25 | 29.4 |
| | % actif/silicone | 62.5 | 25.5 | 70 | 100 | 40 |
| | Rapport co-solvant/silicone | 2 :1 | 3 :1 | 3 :1 | 2 :1 | 2 :1 |

Le taux d'incorporation de la matière active dans le dérivé de silicone varie entre 25,5 % et 100 % suivant sa nature.

Pour l'ensemble des exemples, le rapport pondéral co-solvant/silicone varie de 2 :1 à 3 :1.

### EXEMPLES 6 A 18

### Préparation d'émulsions conformes à l'invention.

On procède à la dispersion du mélange à émulsionner à savoir un concentré préparé selon les exemples 1 à 5 dans de l'eau en présence du Laureth 8OE à titre de tensioactif non ionique sous fort cisaillement.

En tableau 2 ci-après sont présentées les tailles des particules et les concentrations après dilution dans un mélange eau/Laureth sulfate 2OE.

**TABLEAU 2**

| Emulsion | | | Composition | | diamètre |
|---|---|---|---|---|---|
| | Actif | Actif fonction | Mélange utilisé | % final du mélange dans émulsion | µm |
| Exemple 6 | methoxycinnamate d'octyle | Filtre UVB | Exemple 1 | 73 | 0.74 |
| Exemple 7 | methoxycinnamate d'octyle | Filtre UVB | Exemple 1 | 73 | 0.71 |
| Exemple 8 | methoxycinnamate d'octyle | Filtre UVB | Exemple 1 | 73 | 0.71 |
| Exemple 9 | Butyl methoxydibenzoylmethane | Filtre UVA | Exemple 2 | 60 | 0.61 |
| Exemple 10 | Butyl methoxydibenzoylmethane | Filtre UVA | Exemple 2 | 75.8 | 0.64 |
| Exemple 11 | Butyl methoxydibenzoylmethane | Filtre UVA | Exemple 2 | 75.6 | 0.63 |
| Exemple 12 | Octyl methoxycinnamate/Butyl methoxydibenzoylmethane | Mélange Filtre UVB /Filtre UVA | Exemple 3 | 75.2 | 0.7 |
| Exemple 13 | Octyl methoxycinnamateButyl methoxydibenzoylmethane | Mélange Filtre UVB /Filtre UVA | Exemple 3 | 75.2 | 0.65 |
| Exemple 14 | Octyl methoxycinnamate/Butyl methoxydibenzoylmethane | Mélange Filtre UVB /Filtre UVA | Exemple 3 | 75.2 | 0.76 |
| Exemple 15 | Drometrizole trisiloxane | Filtre UVB-UVA | Exemple 4 | 60 | 0.62 |
| Exemple 16 | Drometrizole trisiloxane | Filtre UVB-UVA | Exemple 4 | 60 | 0.70 |
| Exemple 17 | Drometrizole trisiloxane | Filtre UVB-UVA | Exemple 4 | 60 | 0.70 |
| Exemple 18 | Triclosan | Bactéricide | Exemple 5 | 81.8 | 2 |

Les tailles de particules sont de manière générale voisines de 0.7 +/- 0.1 µm.

Les émulsions sont parfaitement stables au stockage. Ainsi, pour les émulsions (exemples 15 à 17), aucune évolution de la taille des particules à l'ambiante sur 9 mois et aucune recristallisation des actifs solides ne sont observées.

Enfin, compte tenu de la composition des mélanges (exemples 1 à 5), le taux d'encapsulation en matière active dans l'huile de silicone varie de 25,5% à 100% selon les actifs.

### EXEMPLES 19 A 22

### Préparation de formulations cosmétiques

L'encapsulation de filtre UVB et/ou UVA et mixte UVB-UVA dans les silicones facilite leur incorporation par exemple dans les formules rincées. De plus, l'utilisation conjointe de polymère cationique et de ces émulsions augmente le dépôt du filtre par exemple du Drometrizole trisiloxane.

L'incorporation d'un mélange (exemples 1 à 5), non émulsionné, conduit, dans une formule destinée au rinçage, à des particules très polydispersées de 15-20 µm et ces grosses tailles sont défavorables au dépôt.

En effet, diverses formulations ont été étudiées à savoir LESNa/CAPB et LESNa/CAMA en présence de polymère cationique.

Les résultats sont présentés en tableau 3 ci-après.

**TABLEAU 3**

| | Composés | Fonction | Exemple 19 | Exemple 20 | Exemple 21 | Exemple 22 |
|---|---|---|---|---|---|---|
| Eau | | Solvant | Qsp 100 | Qsp 100 | Qsp 100 | Qsp 100 |
| Rhéozan ® | Gomme de succinoglycane | Agent épaississant et stabilisant | 0.3 | 0.3 | 0.3 | 0.3 |
| Jaguar Excel ® | Chlorure d'hydroxypropyltria minonium de guar | Agent conditionneur | 0.15 | 0.15 | 0.15 | 0.15 |
| Tegobétaine L7 ® | CocamidopropylBéta ine | Co-tensioactif | - | - | 2.5 | 2.5 |
| Miranol UC32® | Cocoamphoacétate de sodium | Co-tensioactif | 1.2 | 1.2 | - | - |
| Empicol ESB/3M ® | Laureth Sulfate de Tensioactif Laureth Sulfate de sodium | Tensioactif | 10.8 | 10.8 | 10.8 | 7.5 |
| Emulsion Drometrizole Trisiloxane (ex : Exemples 15 à 17) | | | | | | |
| | | | | 1% Filtre | | 1% Filtre |
| | | | | 1% Silicone | | 1% Silicone |
| | | | | | | |
| Mélange | | | 1 % Filtre | | 1% Filtre | |
| (ex : Exemple 4) | | | 1% Silicone | | 1% Silicone | |

| | | | | | | |
|---|---|---|---|---|---|---|
| Le pH est ajusté à 6 | | | | | | |
| NB : Les % sont exprimés en actifs. | | | | | | |

Les rendements de dépôt du Drometrizole trisiloxane (%) en fonction de la taille des particules dans le shampooing sont présentés dans le tableau 4 ci-après.

**TABLEAU 4**

| | Taille des particules dans la formulation | Rendement de dépôt |
|---|---|---|
| | µm | (%) |
| Exemple 19 | 15 | 8+/-2 |
| Exemple 20 | 0,7 | 24 +/- 4 |
| | | |

| | Taille des particules dans la formulation | Rendement de Dépôt |
|---|---|---|
| | µm | (%) |
| Exemple 21 | 15 | 3+/-1 |
| Exemple 22 | 0,7 | 16 +/- 3 |

L'utilisation conjointe d'une émulsion de filtre de faible granulométrie et de polymère cationique est favorable au dépôt comparativement à un mélange synthétique.

Ces émulsions peuvent être utilisées en formule topique.

### EXEMPLE 23

On prépare deux formulations de produits rincés pour le soin de la peau et destinées à conférer une protection solaire après rinçage à l'eau.

Les 2 exemples de formulation donnés ci-après sont deux formulations de type gel douche permettant d'obtenir un SPF in vitro de 4 après rinçage. L'évaluation in vitro de l'indice de protection solaire consiste à étaler une quantité de gel douche définie (2 mg par cm2) sur un support de type Bande Transport® commercialisé par 3M (Sparadrap microperforé hypoallergénique reconnu comme reproduisant le relief de la peau) selon les recommandations de la réglementation européenne pour les produits anti-solaire commercialisés après le 1^{er} janvier 1996 en adéquation avec la méthode COLIPA d'évaluation SPF in vivo. Les mesures de SPF sont réalisées à l'aide d'un Spectrophotomètre UV/Visible, Optometrics SPF 290 Analyser®.

**TABLEAU 5**

| | Formules | Fonction | Exemple 1 | Exemple 2 |
|---|---|---|---|---|
| MIRANOL ULTRA® L32 | Lauroamphoacétate de Sodium | Co-tensioactif | 9.6 | 7.6 |
| EMPICOL ESB/3M | Laureth Sulfate de sodium | Tensioactif | 13 | 10.3 |
| | Acide Isostéarique | | 5 | 4 |
| | Acide citrique | | 1.6 | 1.3 |
| | Emulsion: | | | |
| | Diméthicone de viscosité | | | |
| | 500.000 mPa.s | | 2.3 | 2.3 |
| | Néopentanoate d'isodécyle | | 6.8 | 6.8 |
| | *Filtres UVA+UVB | | 1.6 | 1.6 |
| | Conservateur | | qs | Qs |
| | Eau distillée | | Qsp 100 | Qsp 100 |

| | | | | |
|---|---|---|---|---|
| Note: pH=6-6.5 | | | | |
| *Filtre UVB: Métoxycinnamate d'octyle | | | | |
| *Filtre UVA: Butylméthoxydibenzoylméthane | | | | |

## Revendications

1. Emulsion huile dans l'eau comprenant à titre de phase huileuse au moins un système silicone /co-solvant / .matière(s) active(s), **caractérisé en ce qu'**il comprend sous une forme solubilisée au moins 25% en poids par rapport au poids en silicone d'au moins une matière active liposoluble naturellement non soluble dans ledit silicone.

2. Emulsion selon la revendication 1, **caractérisé en ce que** le co-solvant présente dans l'espace de solubilité de Hansen les paramètres suivants :
δ_{D} d'interactions London variant de 11 à 17 (J/cm³)^{1/2},
δ_{P} d'interactions Keesom supérieur à 2,5 (J/cm³)^{1/2}, et
δ_{H} de liaison hydrogène variant de 0 à 23 (J/cm³)^{1/2}.

3. Emulsion selon la revendication 1 ou 2, **caractérisé en ce que** le silicone présente dans l'espace de solubilité de Hansen les paramètres suivants :
δ_{D} d'interactions London inférieur à 15 (J/cm³)^{½},
δ_{P} d'interactions Keesom inférieur à 1 (J/cm³)^{½}, et
δ_{H} de liaison hydrogène inférieur à 5 (J/cm³)^{½}.

4. Emulsion selon l'une des revendications 1 à 3, **caractérisé en ce que** le silicone possède une viscosité supérieure à 300 mPa.s, de préférence supérieure à 10.000 mPa.s et plus préférentiellement à 100.000 mPa.s.

5. Emulsion selon l'une des revendications précédentes, **caractérisé en ce qu'**il comprend au moins un silicone constitué en tout ou partie de motifs de formule :
R'₃₋ₐRₐSiO_{1/2} et R₂SiO
formules où :
- a est un entier de 0 à 3
- les radicaux R sont identiques ou différents et représentent :
• un groupe hydrocarboné aliphatique saturé ou insaturé contenant de 1 à 10 atomes de carbone ;
• un groupe hydrocarboné aromatique contenant de 6 à 13 atomes
• de carbone ;
• un groupe organique polaire lié au silicium par une liaison Si-C ou Si-O-C ;
• un atome d'hydrogène :
- les radicaux R' sont identiques ou différents et représentent
• un groupe OH
• un groupe alcoxy ou alcényloxy contenant de 1 à 10 atomes de carbone ;
• un groupe aryloxy contenant de 6 à 13 atomes de carbone ;
• un groupe acyloxy contenant de 2 à 13 atomes de carbone ;
• un groupe cétiminoxy contenant de 3 à 8 atomes de carbone ;
• un groupe amino- ou amido-fonctionnel contenant de 1 à 6 atomes de carbone, lié au silicium par une liaison Si-N.

6. Emulsion selon l'une des revendications précédentes, **caractérisé en ce que** le silicone comprend une silicone de type diméthicone ou diphényldiméthicone.

7. Emulsion selon l'une des revendications précédentes, **caractérisé en ce que** le co-solvant est un ester aliphatique d'alkyle en C₁₀ à C₂₀.

8. Emulsion selon l'une des revendications 1 à 6 **caractérisé en ce que** le co-solvant est un néopentanoate d'alkyle en C₅ à C₁₂.

9. Emulsion selon l'une des revendications précédentes, **caractérisé en ce que** le co-solvant est le néopentanoate d'isodécyle.

10. Emulsion selon l'une des revendications précédentes, **caractérisé en ce que** le système comprend du néopentanoate d'isodécyle et un polydiméthylsiloxane de viscosité égale à 500 000 mPa.s.

11. Emulsion selon la revendication 10, **caractérisé en ce que** le co-solvant et le silicone sont associés dans un rapport pondéral variant de 3:2 à 3:1.

12. Emulsion selon l'une des revendications précédentes, **caractérisé en ce que** le système contient de 25 à 100 % en poids en matière(s) active(s) par rapport au poids en silicone.

13. Emulsion selon l'une des revendications précédentes, **caractérisé en ce que** les matières actives liposolubles sont choisies parmi des parfums, des huiles essentielles; des acides gras, des agents colorants, des agents opacifiants, des composés organiques absorbeurs de rayonnement UV, des vitamines, des flavanoïdes, des α- et β-hydroxyacides, des extraits marins, des extraits végétaux, des bactéricides, et leurs mélanges.

14. Utilisation d'une émulsion selon l'une des revendications 1 à 13, pour la préparation d'une émulsion et/ou une formulation concentrée en matière(s) active(s) liposoluble(s).

15. Emulsion selon l'une des revendication 1 à 14, **caractérisée en ce qu'**elle est stabilisée avec un ou plusieurs tensioactifs.

16. Emulsion selon l'une des revendications 1 à 15, **caractérisée en ce qu'**elle est constituée au moins de 50% en poids, et de préférence d'au moins 70% en poids, en un système selon l'une des revendications 1 à 13.

17. Emulsion selon l'une des revendications 1 à 16, **caractérisée en ce qu'**elle présente une taille de gouttelettes comprise entre 25 nm et 350 µm avantageusement inférieure à 3 µm, et plus préférentiellement inférieure à 0,3 µm.

18. Formulation cosmétique, **caractérisée en ce qu'**elle comprend au moins une émulsion selon l'une des revendications 1 à 17.

19. Formulation cosmétique selon la revendication 18, **caractérisée en ce qu'**elle comprend en outre un polymère cationique.

## Claims

1. Oil-in-water emulsion comprising as the oil phase at least one silicone/co-solvent/active material(s) system, **characterised in that** it comprises in dissolved form at least 25% by weight with respect to the weight of silicone of at least one liposoluble active material which is not naturally soluble in the said silicone.

2. Emulsion according to claim 1, **characterised in that** the co-solvent has the following parameters within the Hansen solubility space:
δ_{D} London interactions varying from 11 to 17 (J/cm³)^{1/2},
δ_{P} Keesom interactions over 2.5 (J/cm³)^{1/2}, and
δ_{H} hydrogen bonds varying from 0 to 23 (J/cm³)^{1/2}.

3. Emulsion according to claim 1 or 2, **characterised in that** the silicone present in the Hansen solubility space has the following parameters:
δ_{D} London interactions less than 15 (J/cm³)^{1/2},
δ_{P} Keesom interactions less than 1 (J/cm³)^{1/2}, and
δ_{H} hydrogen bonds less than 5 (J/cm³)^{1/2}.

4. Emulsion according to any one of claims 1 to 3, **characterised in that** the silicone has a viscosity of more than 300 mPa·s, preferably over 10,000 mPa·s, and more preferably 100,000 mPa·s.

5. Emulsion according to any one of the preceding claims, **characterised in that** it comprises at least one silicone wholly or partly comprising units having the formula:
R'₃₋ₐRₐSiO_{1/2} and R₂SiO
in which formulae:
- a is a whole number from 0 to 3
- the R radicals are the same or different and represent:
• a saturated or unsaturated aliphatic hydrocarbon group containing 1 to 10 carbon atoms;
• an aromatic hydrocarbon group containing 6 to 13 carbon atoms;
• a polar organic group linked to the silicon through an Si-C or Si-O-C bond;
• a hydrogen atom:
- the radicals R' are the same or different and represent
• an OH group
• an alkoxy or alkenyloxy group containing 1 to 10 carbon atoms;
• an aryloxy group containing 6 to 13 carbon atoms;
• an acyloxy group containing 2 to 13 carbon atoms;
• a ketiminoxy group containing 3 to 8 carbon atoms;
• an amino or amido functional group containing 1 to 6 carbon atoms linked to the silicon through an Si-N bond.

6. Emulsion according to any one of the preceding claims, **characterised in that** the silicone comprises a silicone of the dimethicone or diphenyldimethicone type.

7. Emulsion according to any one of the preceding claims, **characterised in that** the co-solvent is a C₁₀ to C₂₀ alkyl aliphatic ester.

8. Emulsion according to any one of claims 1 to 6, **characterised in that** the co-solvent is a C₅ to C₁₂ alkyl neopentanoate.

9. Emulsion according to any one of the preceding claims, **characterised in that** the co-solvent is isodecyl neopentanoate.

10. Emulsion according to any one of the preceding claims, **characterised in that** the isodecyl neopentanoate and polydimethylsiloxane system has a viscosity of 500,000 mPa.s.

11. Emulsion according to claim 10, **characterised in that** the co-solvent and the silicone are associated in a ratio by weight varying from 3:2 to 3:1.

12. Emulsion according to any one of the preceding claims, **characterised in that** the system contains 25 to 100% by weight of active material(s) in relation to the weight of silicone.

13. Emulsion according to any one of the preceding claims, **characterised in that** the liposoluble active materials are selected from perfumes, essential oils, fatty acids, colouring agents, opacifiers, organic compounds absorbing UV radiation, vitamins, flavonoids, α- and β-hydroxy acids, marine extracts, plant extracts, bactericides, and their mixtures.

14. Use of an emulsion according to any one of claims 1 to 13 for the preparation of an emulsion and/or a concentrated formulation of liposoluble active material(s).

15. Emulsion according to any one of claims 1 to 14, **characterised in that** it is stabilized with one or more surfactants.

16. Emulsion according to any one of claims 1 to 15, **characterised in that** it comprises at least 50% by weight, and preferably at least 70% by weight, of a system according to any one of claims 1 to 13.

17. Emulsion according to any one of claims 1 to 16, **characterised in that** it has a droplet size of between 25 nm and 350 µm, advantageously less than 3 µm, and more preferably less than 0.3 µm.

18. Cosmetic formulation, **characterised in that** it comprises at least one emulsion according to claims 1 to 17.

19. Cosmetic formulation according to claim 18, **characterised in that** it also comprises a cationic polymer.

## Patentansprüche

1. Öl-in-Wasser-Emulsion, die als ölige Phase mindestens ein Silicon/Colösungsmittel/Wirkstoff(e)-System umfasst, **dadurch gekennzeichnet, dass** es in solubilisierter Form mindestens 25 Gew.-%, bezogen auf das Gewicht des Silicons, mindestens eines fettlöslichen Wirkstoffs, der natürlicherweise in diesem Silicon unlöslich ist, enthält.

2. Emulsion nach Anspruch 1, **dadurch gekennzeichnet, dass** das Colösungsmittel im Hansen-Löslichkeitsraum folgende Parameter besitzt:
- δ_{D} der London-Wechselwirkungen 11 bis 17 (J/cm³)^{1/2},
- δ_{P} der Keesom-Wechselwirkungen von über 2,5 (J/cm³)^{1/2} und
- δ_{H} der Wasserstoffbindung 0 bis 23 (J/cm³)^{1/2}.

3. Emulsion nach Anspruch 1 oder 2, **dadurch gekennzeichnet, dass** das Silicon im Hansen-Löslichkeitsraum folgende Parameter besitzt:
- δ_{D} der London-Wechselwirkungen kleiner als 15 (J/cm³)^{1/2},
- δ_{P} der Keesom-Wechselwirkungen kleiner als 1 (J/cm³)^{1/2} und
- δ_{H} der Wasserstoffbindung kleiner als 5 (J/cm³)^{1/2}.

4. Emulsion nach einem der Ansprüche 1 bis 3, **dadurch gekennzeichnet, dass** das Silicon eine Viskosität von über 300 mPa·s, vorzugsweise über 10 000 mPa·s, und besonders bevorzugt über 100 000 mPa·s besitzt.

5. Emulsion nach einem der vorhergehenden Ansprüche, **dadurch gekennzeichnet, dass** sie mindestens ein Silicon enthält, das ganz oder teilweise aus Grundeinheiten mit der Formel
R'₃₋ₐRₐSiO_{1/2} und R₂SiO
besteht, worin:
- a eine ganze Zahl von 0 bis 3 bedeutet und
- die Reste R gegebenenfalls voneinander verschieden sind und:
• eine 1 bis 10 Kohlenstoffatome enthaltende gesättigte oder ungesättigte aliphatische Kohlenwasserstoffgruppe,
• eine 6 bis 13 Kohlenstoffatome enthaltende aromatische Kohlenwasserstoffgruppe,
• eine polare organische Gruppe, die mit dem Silicium über eine Si-C- bzw. Si-O-C-Bindung verbunden ist, und
• ein Wasserstoffatom bedeuten und
- die Reste R' gegebenenfalls voneinander verschieden sind und:
• eine OH-Gruppe,
• eine 1 bis 10 Kohlenstoffatome enthaltende Alkoxy- oder Alkenyloxygruppe,
• eine 6 bis 13 Kohlenstoffatome enthaltende Aryloxygruppe,
• eine 2 bis 13 Kohlenstoffatome enthaltende Acyloxygruppe,
• eine 3 bis 8 Kohlenstoffatome enthaltende Ketiminoxygruppe und
• eine 1 bis 6 Kohlenstoffatome enthaltende Amino- oder Amido-funktionelle Gruppe, die über eine Si-N-Bindung mit dem Siliciumatom verbunden ist, bedeuten.

6. Emulsion nach einem der vorhergehenden Ansprüche, **dadurch gekennzeichnet, dass** das Silicon ein Silicon vom Typ Dimethicon oder Diphenyldimethicon umfasst.

7. Emulsion nach einem der vorhergehenden Ansprüche, **dadurch gekennzeichnet, dass** das Colösungsmittel ein aliphatischer C₁₀- bis C₂₀-Alkylester ist.

8. Emulsion nach einem der Ansprüche 1 bis 6, **dadurch gekennzeichnet, dass** das Colösungsmittel ein C₅- bis C₁₂-Alkylneopentanoat ist.

9. Emulsion nach einem der vorhergehenden Ansprüche, **dadurch gekennzeichnet, dass** das Colösungsmittel Isodecylneopentanoat ist.

10. Emulsion nach einem der vorhergehenden Ansprüche, **dadurch gekennzeichnet, dass** das System Isodecylneopentanoat und ein Polydimethylsiloxan mit einer Viskosität von 500 000 mPa·s umfasst.

11. Emulsion nach Anspruch 10, **dadurch gekennzeichnet, dass** das Colösungsmittel und das Silicon mit einem Gewichtsverhältnis von 3 : 2 bis 3 : 1 miteinander kombiniert sind.

12. Emulsion nach einem der vorhergehenden Ansprüche, **dadurch gekennzeichnet, dass** das System 25 bis 100 Gew.-% Wirkstoff(e), bezogen auf das Silicongewicht, enthält.

13. Emulsion nach einem der vorhergehenden Ansprüche, **dadurch gekennzeichnet, dass** die fettlöslichen Wirkstoffe aus Parfums, aromatischen Ölen, Fettsäuren, Farbmitteln, opak machenden Mitteln, aus organischen Verbindungen bestehenden UV-Absorbern, Vitaminen, Flavonoiden, α- und β-Hydroxysäuren, marinen Extrakten, Pflanzenextrakten, Bakteriziden und ihren Gemischen ausgewählt sind.

14. Verwendung einer Emulsion nach einem der Ansprüche 1 bis 13 zur Herstellung einer Emulsion und/oder Formulierung, die an fettlöslichem (fettlöslichen) Wirkstoff(en) konzentriert ist/sind.

15. Emulsion nach einem der Ansprüche 1 bis 14, **dadurch gekennzeichnet, dass** sie mit einem oder mehreren Tensiden stabilisiert worden ist.

16. Emulsion nach einem der Ansprüche 1 bis 15, **dadurch gekennzeichnet, dass** sie zu mindestens 50 Gew.-% und vorzugsweise mindestens 70 Gew.-% aus einem System nach einem der Ansprüche 1 bis 13 besteht.

17. Emulsion nach einem der Ansprüche 1 bis 16, **dadurch gekennzeichnet, dass** ihre Tröpfchengröße 25 nm bis 350 µm, vorteilhafterweise weniger als 3 µm, und besonders bevorzugt weniger als 0,3 µm beträgt.

18. Kosmetische Formulierung, **dadurch gekennzeichnet, dass** sie mindestens eine Emulsion nach einem der Ansprüche 1 bis 17 umfasst.

19. Kosmetische Formulierung nach Anspruch 18, **dadurch gekennzeichnet, dass** sie außerdem ein kationisches Polymer umfasst.
